Europäisches Patentamt

European Patent Office

Office européen des brevets

(11) Publication number: 0 345 466
A2

(12) ## EUROPEAN PATENT APPLICATION

(21) Application number: 89108014.5

(22) Date of filing: 03.05.89

(51) Int. Cl.⁴: C12N 15/00

The microorganism has been deposited with American Type Culture Collection under number 33694.

(30) Priority: 10.05.88 US 192089

(43) Date of publication of application:
13.12.89 Bulletin 89/50

(84) Designated Contracting States:
CH DE FR GB LI

(71) Applicant: **EASTMAN KODAK COMPANY (a New Jersey corporation)**
**343 State Street**
**Rochester New York 14650(US)**

(72) Inventor: **Holzschu, Donald Lee c/o Eastman Kodak Company**
**Patent Department 343 State Street**
**Rochester New York 14650 2202(US)**
Inventor: **Daiss, John Linforth c/o Eastman Kodak Company**
**Patent Department 343 State Street**
**Rochester New York 14650 2202(US)**

(74) Representative: **Brandes, Jürgen, Dr.rer.nat. et al**
**Thierschstrasse 8**
**D-8000 München 22(DE)**

(54) **Generation of chimeric antibodies in vivo by site-specific recombination.**

(57) An in vivo method of maxing chimeric antibodies is disclosed. The method comprises the steps of:

a) construction of a transfection plasmid which comprises, in operable association with DNA control regions and selectable markers, a gene segment comprising a gene or gene segment for an immunoglobulin constant heavy or light chain and a gene or gene segment for another function;

b) transfection of the plasmid into a recipient mammalian antibody producing cell that secretes an immunoglobulin having at least seventy (70) percent DNA sequence homology with the immunoglobulin gene or gene segment in the plasmid;

c) screening for the stably transfected antibody producing cells that secrete the chimeric antibody.

FIG. 4

## GENERATION OF CHIMERIC ANTIBODIES IN VIVO BY SITE-SPECIFIC RECOMBINATION

This invention relates to a method of producing chimeric antibodies.

The utility of chimeric antibodies is widely recognized. Chimeric antibodies are currently made from genes constructed by in in vitro methods. These methods are exemplified, for example, by the work of Neuberger et al reported in Nature 312, p 604-608 (1984). These methods generally comprise:

1) preparing a transfer plasmid containing a gene of choice for an antibody heavy chain;

2) preparing a transfer plasmid containing a gene of choice for an antibody light chain;

3) isolating a variable heavy (V$_H$) gene segment from a hybridoma of choice;

4) confirming isolation of the variable heavy gene (V$_H$) of 3);

5) isolating a variable light (V$_L$) gene segment from a hybridoma of choice;

6) confirming isolation of the variable light gene (V$_L$) of 5);

7) inserting the V$_H$ segment into the transfer plasmid of 1);

8) inserting the V$_L$ segment into the transfer plasmid of 2);

9) transfecting both the plasmids of 7) and 8) into a suitable recipient cell; and

10) screening for stable secretors of the chimeric antibody.

This method is labor intensive. It is distinguished by the complete construction of the antibody gene(s) in vitro. It requires the isolation of both the V$_H$ and V$_L$ gene segments for every new chimeric antibody.

Isolation and confirmation of the V$_H$ and V$_L$ gene segments are the most difficult and time-consuming steps of the method. The steps typically require several weeks to several months of work involving a great deal of uncertainty.

The uncertainty and expense necessarily involved in the isolation of variable (V) gene segments causes chimeric antibodies to be extremely expensive. Under these circumstances, only a few widely used chimeric antibodies will be made.

Figure 1 is a schematic of a generalized transfection plasmid used in the method of this invention.

Figure 2 is a schematic of transfection plasmid pKH94 used to illustrate the method of this invention.

Figure 3 is a schematic representation of the method of making a transfection plasmid used in the method of the invention.

Figure 4 is a schematic representation of site-specific recombination.

Many of the problems of time, labor and expense associated with the above-mentioned prior art are overcome by the present invention which comprises the steps:

a) construction of a transfection plasmid which comprises, in operable association with DNA control regions and selectable markers, a gene segment comprising a gene or gene segment for an immunoglobulin constant heavy or light chain and a gene or gene segment for another function;

b) transfection of the plasmid into a recipient antibody producing mammalian cell that secretes an immunoglobulin having. at least seventy (70) percent DNA sequence homology with the immunoglobulin gene or gene segment in the plasmid;

c) screening for the stably transfected antibody producing mammalian cells that secrete the chimeric antibody.

The recipient cell is a mammalian antibody producing cell, including myelomas, hybridomas and B-cell lines. In an especially useful embodiment of the invention, the recipient antibody producing mammalian cell is a hybridoma, particularly a mouse hybridoma, that secretes an immunoglobulin of the same class as the immunoglobulin gene segment in the transfection plasmid.

The "another function" is achieved by the addition and/or deletion of gene(s) or gene fragment(s), to add or delete functions such as enzymatic, ligand binding, stop codons, directed glycosylation, altered immunogenecity, etc.

Stable transfected cells are those which survive after thirty days in culture.

A "gene or gene segment" includes situations in which all or part of an immunoglobulin or new function is added or deleted in the transfection plasmid.

This method does not require the isolation of V$_H$ or V$_L$ gene segments. In the method of this invention the gene for the chimeric antibody is assembled inside the recipient antibody producing mammalian cell. Consequently the method is "in vivo". The chimeric gene is created in vivo by insertion of an exogenous genetic element, in the form of a transfection plasmid, directly into the active immunoglobulin heavy or light chain gene of the antibody producing cell by a process called targeted or site-specific recombination.

By virtue of the homology between the immunoglobulin portion of the immunoglobulin gene in the plasmid and the active antibody gene in the recipient cell the plasmid is inserted by the cell's own "genetic machinery" directly into its active heavy or light chain gene yielding:

i) an active heavy or light chain gene which produces a chimeric antibody; and

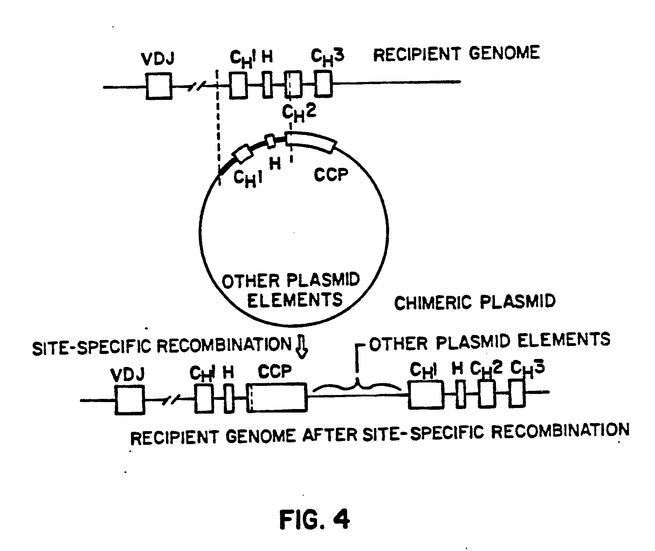

ii) ideally, a characteristic insertion of the plasmid will have the heavy or light chain gene on the 5'-end, a constant region gene on the 3'-end and the other transfection plasmid elements in between (see figure 4).

The in vivo method for creating chimeric antibodies has three principle advantages over the widely practiced in vitro method:

i) no variable region gene segment needs to be isolated;

ii) once a transfection plasmid is constructed that same plasmid can be used without modification to create similar chimeric antibodies of any specificity so long as the heavy or light chain antibody class of the recipient antibody producing cell is sufficiently homologous as defined herein, with the heavy or light chain immunoglobulin gene included in the plasmid; and

iii) the recipient cell for the plasmid is the cell which secretes the antibody of the chosen specificity.

These practical advantages translate into a simpler, faster, less expensive method of making chimeric antibodies.

The in vivo method has three elemental steps:

i) construction of a transfection plasmid chimeric gene segment consisting of the antibody constant heavy or light gene segment and the gene for the new function;

ii) transfection of a suitable recipient hybridoma cell with the transfection plasmid; and

iii) screening for the stably transfected mammalian antibody producing cell that secretes the chimeric antibody.

## 1. General Structure of the Transfection Plasmid

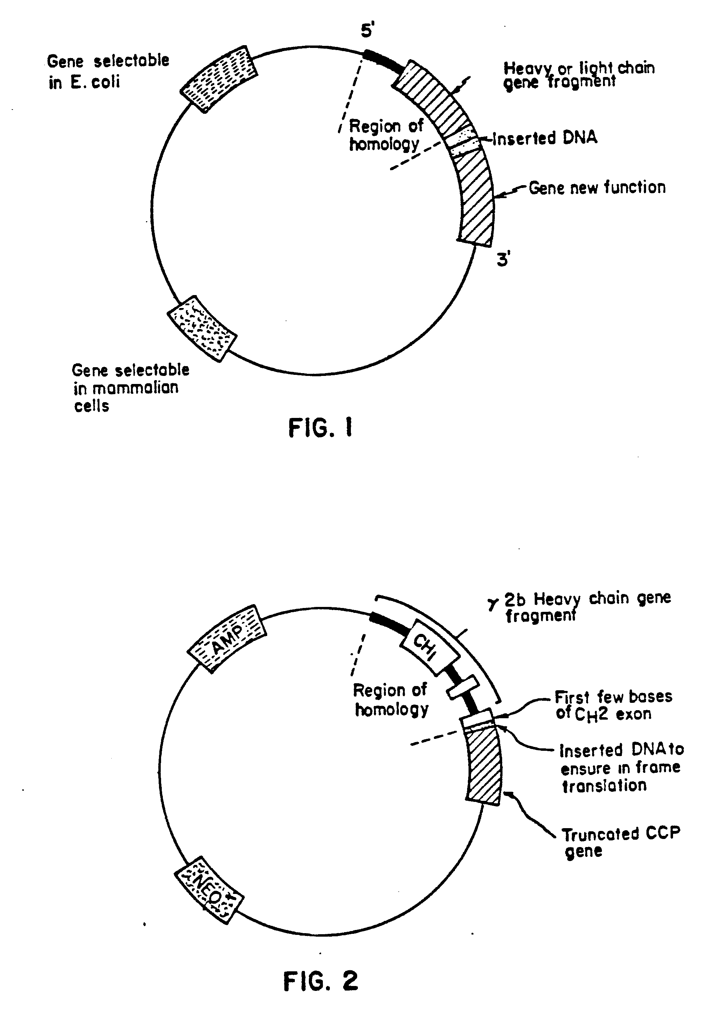

A suitable transfection plasmid necessarily contains five parts as shown, in part, in figure 1. The first part consists of genetic markers for selectable cultivation in E.coli. The second is a selectable marker for mammalian cells. Several such plasmids have been developed and are widely used; pSV2neo and pSV2gpt are examples. The third part is a segment for an immunoglobulin constant heavy or light chain gene sufficiently large to allow for site-specific recombination. The fourth part is the gene segment for the new function one wishes to insert. Examples of such functions include enzymes such as horseradish peroxidase or alkaline phosphatase, binding proteins such as avidin or metallothionein and immunoglobulin gene segments. The entire gene may not be necessary. The fifth part (not shown) comprises the gene segments that encode regulatory elements, termination of transcription or polyadenylation sites, splice sites or linkers which may have to be deleted or added. In the construction, small DNA segments may be added or deleted to keep the assembled gene in translational frame. The essential elements are generally assembled as shown in figure 1.

In order for the appropriate heavy or light chain gene to form in vivo, the plasmid must be transfected into a recipient hybridoma cell. Transfection can be accomplished by any of a number of methods including osmotic shock, calcium phosphate precipitation, liposome or protoplast fusion, or electroporation. Transfected hybridoma cells are cells which have incorporated the plasmid into their genomes as indicated by a selectable marker. They can be identified by growing the cells in medium selectively toxic to non-transfected cells. Typically, about one cell in 10,000 is stably transfected in electroporation experiments.

The population of stably transfected cells contains two subpopulations: 1) those which have incorporated the plasmid at an irrelevant site in their genomes and still secrete the normal antibody; and 2) those that have incorporated the plasmid into their active heavy chain gene and are secreting a chimeric antibody. These cells can then be screened by standard enzyme-linked immunoassay methods (ELISA) for the rare cells secreting the chimeric antibody. In principle one can screen for three distinct properties:

i) the functional activity of the new protein;

ii) the antigenic activity of the new protein; or

iii) the loss of epitopes normally detectable in the deleted portion of the heavy or light chains.

Cells secreting chimeric antibodies are then cloned and cultivated by standard methods in order to obtain significant quantities of the chimeric antibody for further purification, characterization or use.

## 2. Transfection Plasmid (pKH94)

The specific plasmid used herein to transfect the selected hybridoma and illustrate the invention is presented schematically in figure 2. It was used to induce the production of chimeric antibodies in the selected hybridoma. The vector has the following operably linked genetic components. The acronyms or names in parenthesis following each component are the symbols used for each component in figures 1 and 4.

A. A gene or gene segment for any new function which is to be associated with the antibody thereby making the antibody chimeric. Examples of such new functions are mentioned hereinbefore. To illustrate the invention a gene 4 for cytochrome c peroxidase (CCP) was used in pKH94.

B. An E.coli origin of replication (not shown). This enables the intermediate and final plasmids (pKH94) to replicate in E.coli.

C. A β-lactamase gene (AMP). This is a gene that codes for β-lactamase which confers ampicillin resistance to E.coli. Any E.coli drug resistance gene known in the art will also be useful. Drug resistance provides a means of selecting for the growth of cells containing plasmids having the drug resistance.

D. Tn5 aminoglycoside phosphotransferase gene (neo). Neo is a gene that confers resistance to the antibiotic G418 in mammalian cells. It is useful in selecting the hybridoma cells which have been successfully transfected with pKH94. Other selectable genes, in operable association with suitable promotors and terminators, are similarly useful, e.g., dihydrofolate reductase and E.coli guanosine phosphoribosyl transferase.

E. A gene or gene segment for an immunoglobulin heavy chain. The gene is selected in this embodiment to match the heavy chain normally secreted by a selected hybridoma. In this case a segment of immunoglobulin γ2b constant gene region was used to exemplify the invention. The segment is 2.1 kb of the mouse γ2b heavy chain gene starting from 1.3 kb 5' of the $C_H1$ exon obtained from Dr. Michael Neuberger from Medical Research Council, England, in a plasmid called pSV-V$_{np}$γSNase. This gene segment is in translational frame with the CCP gene.

## 3. General Method of Making pKH94

The transfection vector, pKH94, was made using standard recombinant DNA methods. pKH94 was constructed from a combination of four already existing plasmids: pSV2neo containing the bla and neo genes; pSV-V$_{np}$γSNase containing the γ2b gene segment; pUC18 containing a multiple cloning site and pKH74 containing the CCP gene. The DNA gene expression control elements were also included in pSV2neo ( ATCC No.: 37 149 ).

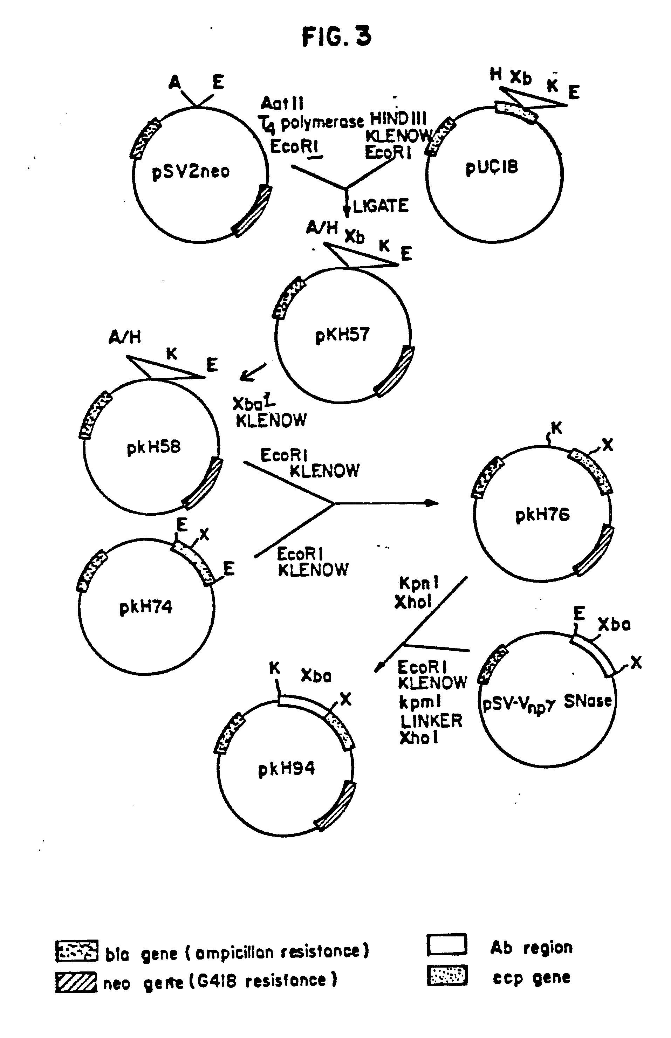

Figure 3 is a schematic illustration of the various steps of the method for making pKH94. In general, the method involved:

A. removing the multiple cloning site from pUC18;

B. inserting the multiple cloning site into pSV2neo to form intermediate plasmid (pKH57);

C. removing a Xba1 restriction site from the multiple cloning site of pKH57 to make pKH58;

D. removing the CCP gene from pKH74;

E. inserting the CCP gene into the multiple cloning site of pKH58 to form intermediate plasmid pKH76;

F. removing the γ2b gene segment from pSV-V$_{np}$γSNase; and

G. inserting γ2b in pKH76 in frame with the CCP gene in pKH76 resulting in pKH94.

## 4. Detailed Method of Making pKH94

### Construction of pKH57 (figure 3, step 1)

#### A. Reagents

.   All restriction enzymes, DNA polymerase large fragment (Klenow), T4 DNA ligase, and T4 DNA polymerase were from New England Biolabs and used according to vendor specifications. Kpn1 linkers were purchased from New England Biolabs. Ampicillin and Geneticin (G418) were purchased from Gibco.

#### B. Bacterial Strains and Plasmids

E.coli strain HB101 (F⁻leuB6, proA2, recA13, thi-1, ara-14, lacY1, galK2, xyl-5, mtl-1, rpsL20, λ⁻, supE44, hsdS20r⁻$_B$m⁻$_B$) was used to propagate plasmids and as a transfection host.

The plasmid pSV2neo, from ATCC, contains the gene encoding ampicillin resistance for selection in E.coli; the neo gene encoding G418 resistance for selection in mammalian cells and all of the necessary DNA gene expression elements. The plasmid pKH74 was used as the source of the cytochrome c peroxidase gene. The preparation thereof is described in USSN 109,796 filed October 16, 1987, in the name of Ciccarelli et al and is expressly incorporated herein by reference. The immunoglobulin γ2b constant region segment was subcloned from pSV-V$_{np}$γSNase, which was obtained from Dr. Michael Neuberger, is described in Nature 312, p 604-608 (1984).

#### C. Methods

All standard recombinant plasmid manipulations, such as ligation, filling of cohesive DNA ends, digestion with restriction enzymes, DNA sequencing, restriction mapping and large scale preparation were done according to Maniatis et al, Molecular Cloning: A Laboratory Manual, Cold Spring Harbor Laboratory, New York, 1982.


D. Plasmid Construction

The plasmid used to transfect mouse hybridoma cells, pKH94, contains a segment of the immunoglobulin $\gamma$2b constant gene region fused in translational frame to the yeast CCP gene. pKH94 was constructed as shown in figure 3.

The multiple cloning site (MCS) of pUC18 was subcloned into pSV2neo to provide useful restriction sites. To remove the multiple cloning site (MCS); pUC18 was digested with Hind111; the resulting cohesive ends filled using Klenow fragment and dNTPs then digested with EcoR1.

pSV2neo was digested with Aat11; the cohesive ends filled using T4 DNA polymerase and dNTPs; and then digested with EcoR1.

These preparations of pSV2neo and pUC18 were mixed together, ligated and used to transfect E.coli HB101 to ampicillin resistance. Selected plasmids were analyzed by restriction analysis. pKH57, combining pSV2neo and the pUC18 MCS, was recognized by containing a unique Xba1 site, a unique Kpn1 site, two BamH1 sites, a unique Hind111 site, and an appropriate sa11 site.

The multiple cloning site (MCS) in pKH57 includes an Xba1 (X) restriction site. $\gamma$2b also includes an Xba1 site which in our construction strategy was used to confirm the presence of $\gamma$2b in pKH94. Therefore, it was necessary to remove Xba1 from the multiple cloning site. To destroy the Xba1 site in pKH57, the plasmid was digested with Xba1. The resulting cohesive ends were made blunt as hereinbefore. The plasmid was recircularized by ligation. After ligation the solution containing pKH57 was again digested with Xba1 to eliminate any plasmids that still contained the Xba1 site. Plasmids not cut by Xba1 were designated pKH58.

The cytochrome c peroxidase (CCP) gene from pkH74 was prepared for ligation into pKH58 by cutting pKH74 with EcoR1 to remove the CCP gene. The cohesive ends of the removed gene were filled using Klenow fragment. The cytochrome c peroxidase gene in pKH74 had been previously modified by: 1) deletion of the CCP gene segment encoding the leader peptide of pre-CCP, 2) insertion of an Xho1 site 6 bp 5′ to the start of translation, and 3) insertion of an additional adenine residue between the Xho1 site and the start of CCP translation. See figure 1, inserted DNA. The additional adenine residue was added to put the CCP segment into translational frame with the $\gamma$2b at the Xho1 splice site in pKH76. pKH58 was cut with EcoR1 and the ends filled as above. The CCP gene was then ligated into pKH58 as indicated in figure 1 to form intermediate plasmid pKH76. Following ligation and cloning in E.coli, clones were analyzed for the presence of the CCP gene segment. One plasmid, designated pKH76 was identified as containing the gene and saved for further manipulations.

The $\gamma$2b gene segment was obtained from the plasmid pSV-$V_{np}\gamma$SNase. pSV-$V_{np}\gamma$SNase was digested with EcoR1. The ends were blunted using Klenow fragment and dNTPS. A Kpn1 linker was added to the blunted ends. The plasmid was then digested with Xho1 to produce a Kpn1-Xho1 $\gamma$2b fragment between the added Kpn1 (K) site and the Xho1 (X) of approximately 2.1 kb. pKH76 was cut with Kpn1 and Xho1. The $\gamma$2b fragment was ligated into pKH76 to produce pKH94. The construction of pKH94 was confirmed by restriction mapping analysis. pKH94 has the specific construction shown in figure 2.


5. Site-Specific Recombination (Figure 4)

The chimeric antibody encoding genes made according to the in vivo method described in this invention are created by site-specific recombination. Site-specific recombination occurs between a recipient hybridoma genome and an exogenous genetic element, such as the transfection plasmid of figure 2, if there is a significant region of homology in the recipient genome and the transfection plasmid.

Recombination in mammalian cells has been shown to occur between DNA segments with an overall DNA sequence homology of seventy percent (70%) or higher. We believe that a plasmid comprising a constant region gene segment could specifically integrate into other constant region loci which have at least 70% overall sequence homology.

A region of homology is denoted by the dashed lines in figures 2 and 4. The region of homology (figures 2 and 4) is a 2.1 kb segment of the $\gamma$2b heavy chain gene that runs from about 1.3 kb 5′ of the $C_H1$ domain-encoding exon to 15 bp into the $C_H2$ domain-encoding exon.

The characteristic product of the recombination event is illustrated at the bottom of figure 4. Essentially, the plasmid is inserted into the recipient genome somewhere in the region of homology yielding two copies of the region of homology. The two copies are separated by the other plasmid elements. In figure 4 the region of homology extends from the intron 5' to the $\gamma$-2b$C_H$1 domain-encoding exon to just a few nucleotides into the $\gamma$2b-$C_H$2 domain-encoding exon. The CCP was incorporated into the plasmid immediately 3' to the region of homology. As a result of the recombination event, the CCP gene segment is substituted for the normal gene $\gamma$2b segments which are displaced (see figure 4). This was accomplished by the methods that follow.

## 6. Transfection and Screening

### A. Recipient Antibody Producing Mammalian Cell

A hybridoma cell line Phe 4.5, was used as the recipient cell to illustrate the invention. Phe 4.5 cell line is the product of a fusion between SP2/O-Ag14 myeloma cells (Shulman et al, Nature 276, 269-270, 1978) and lymphocytes from a Balb/c mouse immunized with phenobarbital-conjugated Bovine Serum Albumin. The immunoglobulin product of Phe 4.5 has a nominal specificity for phenobarbital (Ka<$10^6$) and binds readily to phenobarbital-conjugated glucose oxidase (Phe-GOD). Phe 4.5 has an antibody isotype $\gamma$2b,$x$.

### B. Transfection

#### i) Preparation of Plasmid

Plasmid pKH94 was transformed into E.coli strain HB101. From cultures of these cells the plasmid was prepared in milligram quantities essentially according to Maniatis et al, Molecular Cloning: A Laboratory Manual, Cold Spring Harbor Laboratory, New York, 1982. Transfected HB101 bearing pKH94 was cultivated in the presence of chloramphenicol to amplify the plasmid. Transfected HB101 was then lysed and the plasmid was extracted after treating the lysate with RNAaseA and sedimenting cellular debris. The plasmid was then purified from the extract by equilibrium sedimentation on a cesium chloride gradient.

The day before transfection, the circular plasmid pKH94 was linearized by cleavage at the unique Xba1 site in the $\gamma$2b gene segment ~0.8 kb 5' from the protein coding sequence. The linearized plasmid was stored as a precipitate in 95% EtoH at -20° C and was redissolved in PBS the day of the transfection.

#### ii) Preparation of Recipient Cells

Phe 4.5 cells were maintained in T-150 flasks in DMEM supplemented with 10% FCS under standard cell culture conditions (5% $CO_2$ in air, 100% RH, 37° C, dark). They were passed every other day until 24 hours prior to transfection when they were split 1:2 with fresh medium and seeded into fresh T-150 flasks. This step ensured that the cells were in log phase for the transfection. Immediately before transfection the Phe 4.5 cells were washed twice with PBS and then resuspended in PBS.

#### iii) Electroporation

100 $\mu$l Phe 4.5 cells (2.7x $10^7$ cells/ml) and 100 $\mu$l purified pKH94 (80 $\mu$g/ml) were mixed and placed between two tracks of a five-channel electrofusion chamber (Biolectronics Inc., Troy, Michigan). After a 30 $\mu$sec, 2000V pulse from a High Voltage Cell processor (Biolectronics) the cells were suspended in 20 ml DMEM supplemented with 20% FCS and distributed into two 96-well microculture plates (2.7x $10^4$ cells/well). The cells were then allowed to grow without selection for three days.

### C. Screening

After three days culture without selection, the culture medium was exchanged for DMEM supplemented with 20% FCS and 1 mg/ml G418 (Gibco). G418 is a drug which selects for cells expressing TnS aminoglycoside phosphotransferase (NEO) which is the selectable marker for pKH94. Fresh G418 containing medium was added every third day. By the thirtieth day one or more macroscopic colonies was visible in most wells. The culture supernatants were ready for screening to identify the hybridomas secreting the desired chimeric antibodies.

#### i) Immunochemical Reagents

a) Antisera to Cytochrome C Peroxidase (anti-CCP) was prepared as follows. New Zealand albino rabbits were immunized with 1 mg CCP in Complete Freund's Adjuvant at eight subcutaneous sites. Animals were boosted at four-week intervals and serum was collected. The antiserum had a titer of 1:16 in radial immunodiffusion against 1 mg/ml CCP and its titer exceeded 1:100,000 in ELISA tests for native CCP. Affinity-purified anti-CCP antibody was prepared by purification on CCP-Sepharose made by the method of Cuatrecasas, J. Biol. Chem. 245, 3059 (1970).

b) HB58 is a rat-mouse hybridoma which secretes an IgG1 rat antibody specific for mouse kappa light chains. It was generated by Scharff et al, Hybridoma 1, 1-5 (1981) and obtained from ATCC. Milligram quantities were purified from shake flask cultures essentially by the method of Ey et al, Immunochemistry 15, 429-436 (1978).

c) Horseradish Peroxidase (HRP) Conjugation. Affinity purified rabbit anti-CCP or purified HB58 monoclonal antibody were conjugated with HRP by the method of Nakane and Kawasi, J. Histochem. Cytochem. 22, 1084-1091 (1974).

d) Horseradish peroxidase conjugated goat anti-mouse γ2a/γ2b was obtained from Jackson Immunoresearch.

e) Phenobarbital valerate glucose oxidase (Phe-GOD) was prepared by the method of Erlanger, J. Biol. Chem. 234, 1090 (1959).

## ii) Assay for CCP Antigenic Determinants

The chimeric antibody, if secreted, would have the CCP gene in the Fc portion of the antibody. Phe-GOD (2 μg/ml in PBS) was adsorbed onto the wells of flat-bottomed polystyrene microtiter plates and residual surface was blocked with BSA. 100 μl samples of culture supernatant were added to corresponding wells. After a one hour incubation at room temperature (RT) plates were washed and HRP-conjugated rabbit anti-CCP antibody was added and incubated for one hour at RT. After washing, the retained peroxidase activity was assayed.

## iii) Assay for the Presence of Fab and Absence of Fc Determinants

Samples of culture supernatants were incubated with 100 μl of a 20% suspension of Pansorbin (Calbiochem) in PBS in 96 well microtiter plate in order to remove Fc-bearing molecules. The Pansorbin was then pelleted by centrifugation and the supernatant was split into separate ELISA assays for Fab and Fc. To test for Fab, culture supernatants were incubated on microtiter plates coated with Phe-GOD. After washing, each sample was scored with HRP-HB58 (monoclonal rat anti-mouse kappa chain). To test for Fc, culture supernatants were incubated on plates coated with Phe-GOD and scored with HRP-conjugated goat anti-mouse γ2a/b. Samples that scored Fab+ and Fc− were cloned in soft agar and then cultivated to prepare material for further characterization.

## 7. Purification and Characterization of Chimeric Antibody

## A. Purification by Affinity Chromatography

Immobilized phases for affinity chromatography on cyanogen-bromide-activated sepharose essentially according to Cuatrecasas, J. Biol. Chem. 245, 3059 (1970). HB58 was immobilized as a ligand for any kappa chain-bearing molecules. Affinity purified rabbit anti-CCP antibody was immobilized as a ligand for any CCP bearing molecules. All separations were prepared in essentially the same manner.

## B. Gel Electrophoresis

Sodium Dodecyl Sulfate-Polyacrylamide Gel Electrophoresis (SDS-PAGE). SDS-PAGE was performed in slab gels essentially by the method of Laemmli (Nature 227, 680, 1970).

## C. Immunoblotting

Freshly run SDS-PAGE gels were equilibrated in transfer buffer and the electroblotted onto nitrocellulose using a BioRad Transblot (90 min, 70V). Blots were labeled and blocked by overnight incubation in PBS with 1% BSA. Blotted samples were then incubated for 2 hours with appropriate dilutions of HRP-conjugated antibodies in 1% BSA washed extensively with PBS and then developed for HRP activity with 4-chloronaphthol.

## 8. Biochemical and Immunochemical Characterization

In the above screens and characterizations, antibodies were identified that bound to Phe-GOD, had CCP antigenic activity and lacked Fc antigenic activity. Further characterization of these chimeric molecules demonstrated that they had a number of properties characteristic of the anticipated chimeric antibody. Specifically, the chimeric antibody molecules secreted by these cells:

i) have heavy chains larger than the parental IgG2b heavy chain (~65 kd compared to ~55 kd) as predicted by the difference between the mass subtracted from the γ2b gene and that added by the CCP gene;

ii) have covalently bound kappa light chains and can be purified by affinity chromatography on immobilized anti-kappa antibodies;

iii) have CCP antigenic determinants as demonstrated by both immunoblotting and purification by affinity chromatography on immobilized anti-CCP antibodies; and

iv) can be fragmented by limited proteolysis with V8 protease from Staphylococcus aureus to yield anti-CCP reactive fragments identical in mass to several generated from native CCP.

The foregoing detailed construction and ELISA screening tests demonstrate that the in vivo method of this invention is useful in generating chimeric antibodies. The generation of chimeric antibodies with any of a number of protein segments is possible.

Instead of inserting a "gene" for a new protein in place of the $C_H$ gene segments a specifically designed synthetic oligonucleotide could be inserted. Such oligonucleotides might encode sites for selective covalent modification (e.g., glycosylation) or stop codons.

## Claims

1. A method of making chimeric antibodies comprising the steps of:

a) construction of a transfection plasmid which comprises, in operable association with DNA control regions and selectable markers, a gene segment comprising a gene or gene segment for an immunoglobulin constant heavy or light chain and a gene or gene segment for another function;

b) transfection of the plasmid into a recipient mammalian antibody producing cell that secretes an immunoglobulin having at least seventy (70) percent DNA sequence homology with the immunoglobulin gene or gene segment in the plasmid;

c) screening for the stably transfected antibody producing cells that secrete the chimeric antibody.

2. The method of claim 1 wherein the recipient antibody producing cell is a hybridoma that secretes an immunoglobulin of the same chain class as the immunoglobulin gene or gene segment included in the plasmid.

3. The method of claim 1 or 2 wherein the antibody gene segment comprises a gene or gene segment for an immunoglobulin constant heavy chain ($C_H$).

4. The method of claim 1 or 2 wherein the chimeric gene segment comprises a gene or a gene segment for immunoglobulin constant light chain ($C_L$).

5. The method of claim 1, 2, 3 or 4 wherein the another function is selected from gene or gene segments coding for enzymatic, ligand binding, stop codons, directed glycosylation and altered immunogenecity functions.

6. The method of claim 1 or 2 wherein transfection is carried out by osmotic shock, calcium phosphate precipitation, liposome or protoplast fusion, or electroporation.

7. The method of claim 2 wherein the chain class is selected from the group consisting Igm, IgG, IgA, IgE and IgD of any mammalian antibody producing cell.

8. The method of claim 1 wherein the recipient antibody producing cell is a myeloma cell line or a B-cell line.

Gene selectable in E. coli

5'

Heavy or light chain gene fragment

Region of homology

Inserted DNA

Gene new function

3'

Gene selectable in mammalian cells

FIG. 1

AMP

γ 2b Heavy chain gene fragment

CH1

Region of homology

First few bases of $C_H2$ exon

Inserted DNA to ensure in frame translation

Truncated CCP gene

NEO

FIG. 2

# FIG. 3

bla gene (ampicillon resistance)   Ab region

neo gene (G418 resistance)   ccp gene

FIG. 4